# FASCICULE DE BREVET EUROPEEN

(11) **EP 4 054 426 B1**
(45) Date de publication et mention de la délivrance du brevet: **28.01.2026**
(21) Numéro de dépôt: 20800665.0
(22) Date de dépôt: 06.11.2020
(51) Int. Cl.: A61B 17/00, A61L 31/00, A61P 17/02, A61K 35/51

(54) **DISPOSITIF DE COMBLEMENT DE FISTULE**
FISTELFÜLLVORRICHTUNG
FISTULA FILLING DEVICE

(30) Priorité: 06.11.2019 FR 1912473
(43) Date de publication de la demande: 14.09.2022
(73) Titulaire: TBF Genie Tissulaire (TBF), 69780 Mions (FR)
(72) Inventeur: BARNOUIN, Laurence, 38460 VENERIEU (FR)
(74) Mandataire: Tripoz, Inès
(86) Numéro de dépôt international: PCT/EP2020/081350
(87) Numéro de publication internationale: WO 2021/089820

(56) Documents cités:
- WO-A1-2017/100166
- FR-A1- 3 058 321
- US-A1- 2008 245 374
- US-A1- 2009 125 119
- US-A1- 2009 326 577

## Description

L'invention concerne un dispositif de comblement de fistule.

L'invention concerne également un procédé de fabrication dudit dispositif de comblement de fistule.

### Art antérieur

Les fistules sont des passages anormaux qui se créent entre deux organes internes du corps humain à la suite par exemple d'une infection, d'un traumatisme, d'une maladie congénitale, de la maladie de Crohn, d'effet secondaire d'une chirurgie.

Les fistules peuvent être de différents types selon les organes qu'elles relient : ano-rectales, urètro-vaginales, vesico-vaginales, recto-vaginales, trachéo-esophagiques, gastro-cutanées, recto-vésicales, recto-urètrales, recto-prostatiques.

Une des méthodes de traitement d'une fistule consiste à la combler par injection d'une colle biologique ou insertion d'un dispositif de comblement biocompatible.

Le document WO2006119256 divulgue une greffe médicale sous forme d'une feuille de matériau biocompatible remodelable enroulée pour le traitement de fistules.

Le document WO2007002260 divulgue une greffe implantable pour combler une fistule en matériau biologique issu d'un donneur humain mort ou d'un donneur non humain, comprenant un corps sous forme de bouchon ainsi qu'un capuchon.

Le document WO2012050836 divulgue un dispositif pour le traitement de fistules sous forme de bouchon comprenant une pluralité de feuilles de matériau biologique issues de greffes de donneurs.

Le document WO2013009281 divulgue une prothèse implantable pour réparer une fistule en matériau biocompatible comprenant un canal permettant le drainage de la fistule.

Le document WO2017100166 divulgue un dispositif pour traiter les blessures profondes ou sous forme de tunnel incluant les fistules, ledit dispositif comprenant une partie centrale allongée constituée d'une matrice tissulaire, ainsi qu'un groupe de corps formés de matrice tissulaire possédant des ouvertures permettant de laisser passer une portion de la partie centrale allongée, ces corps pouvant être constitués d'un matériau de matrice cellulaire coupé ou micronisé.

Le document US2009125119 divulgue un dispositif médical de greffe comprenant un capuchon et un corps sous forme de bouchant allongé s'étendant depuis le capuchon, le capuchon pouvant comprendre un manchon s'étendant depuis une partie centrale de sa partie basse.

Le document US2008245374 divulgue un bouchon de fistule comprenant un corps allongé et pouvant comprendre un matériau de cœur amovible contenu dans le corps dudit bouchon.

Le document US2009326577 divulgue des constructions de greffes volumétriques constituées de matériaux de matrice extracellulaire ou de matériaux de matrice extracellulaire modifiés physiquement.

### Description des figures

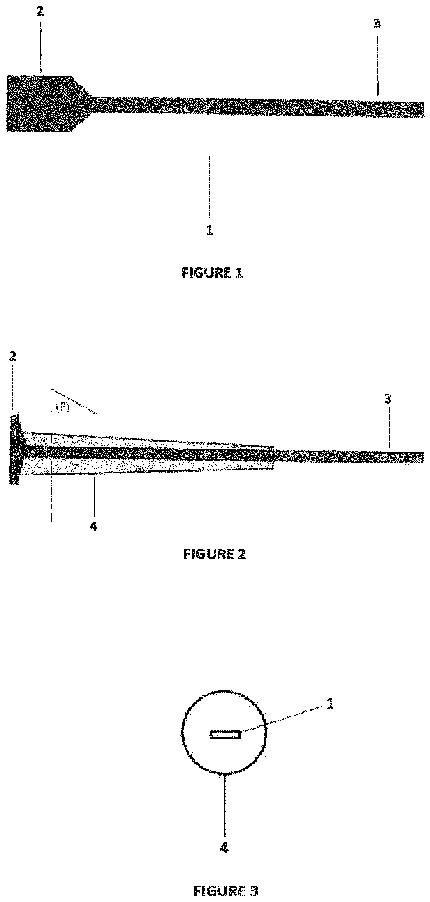

La figure 1 illustre un matériau biocompatible sous forme d'une languette (1) comprenant une extrémité proximale (2) et une extrémité distale (3).
La figure 2 illustre un dispositif selon l'invention comprenant ledit matériau biocompatible sous forme d'une languette (1) entouré par le matériau biologique issu de cordon ombilical (en transparence) sous forme d'un broyat moulé et formant un manchon (4).
La figure 3 illustre la section d'un dispositif selon l'invention selon le plan (P).

### Description détaillée de l'invention

La présente invention concerne un dispositif de comblement de fistule comprenant :
- un matériau biocompatible sous forme d'une languette (1) ayant une extrémité proximale (2) et une extrémité distale (3) ;
- un matériau biologique sous forme d'un broyat moulé entourant le matériau biocompatible et formant un manchon (4) ;
caractérisé en ce que ledit matériau biologique est issu de cordon ombilical.

On entend par « matériau biocompatible, tout matériau compatible avec l'insertion dans un organisme vivant. Cette définition inclut les matériaux biologiques.

On entend par « matériau biologique », tout matériau issu de tissus humains, animaux ou végétaux.

On entend par « languette », un objet qui a une forme mince, étroite et allongée.

On entend par « manchon », une pièce creuse en forme de fourreau ou de gaine susceptible par enveloppement d'entourer une autre pièce ou un élément de forme généralement allongée ; le manchon peut être un manchon à section circulaire, un manchon à section carrée, un manchon à section triangulaire, un manchon à section rectangulaire etc.

Dans un mode de réalisation, le dispositif selon l'invention est caractérisé en ce que le matériau biocompatible sous forme d'une languette (1) possède une longueur supérieure à celle manchon (4).

Dans un mode de réalisation, le dispositif selon l'invention est caractérisé en ce que l'extrémité proximale (2) de ladite languette (1) n'est pas entourée par le manchon (4).

Dans un mode de réalisation, le dispositif selon l'invention est caractérisé en ce que l'extrémité proximale (2) de ladite languette (1) est plus large que l'extrémité distale (3).

Dans un mode de réalisation, le dispositif selon l'invention est caractérisé en ce que l'extrémité proximale (2) de ladite languette (1) est de forme géométrique choisie dans le groupe constitué des formes de carré, de triangle, de cercle, de rectangle, d'octogone, de losange, de trapèze, d'ovale, d'ellipse, de pentagone et d'hexagone.

L'extrémité proximale (2) de ladite languette (1) a pour but de fixer le dispositif à la paroi du tissu dans lequel est situé l'extrémité de la fistule, afin que celui-ci reste en position dans la fistule. L'extrémité proximale (2) de ladite languette (1) peut être fixée à l'aide d'une colle biologique ou de points de sutures. La fixation a également pour but d'obstruer l'une des deux entrées de la fistule.

Dans un mode de réalisation, le dispositif selon l'invention est caractérisé en ce que l'extrémité distale (3) de ladite languette (1) n'est pas entourée du manchon (4).

L'extrémité distale (3) de ladite languette (1) a pour but de faciliter la préhension et le guidage lors de l'insertion du dispositif dans la fistule. L'extrémité distale (3) de ladite languette (1) permet également le drainage de la fistule.

Le matériau biocompatible sous forme d'une languette (1) aura une longueur adaptée selon l'emplacement et la longueur de la fistule à combler.

Dans un mode de réalisation, le dispositif selon l'invention est caractérisé en ce que le matériau biocompatible sous forme d'une languette (1) a une longueur comprise entre 1 et 20 cm, de préférence entre 2 et 10 cm.

Dans un mode de réalisation, le dispositif selon l'invention est caractérisé en ce que le matériau biocompatible sous forme d'une languette (1) est flexible.

Dans un mode de réalisation, le dispositif selon l'invention est caractérisé en ce que le matériau biocompatible sous forme d'une languette (1) est incurvé.

On entend par « incurvé », courbé de façon à former un arc de cercle.

Dans un mode de réalisation, le dispositif selon l'invention est caractérisé en ce que ledit matériau biocompatible sous forme d'une languette (1) est un matériau biologique.

Dans un mode de réalisation, le dispositif selon l'invention est caractérisé en ce que ledit matériau biocompatible sous forme d'une languette (1) est un matériau biologique issu de cordon ombilical.

Dans un mode de réalisation, le dispositif selon l'invention est caractérisé en ce que ledit matériau biocompatible sous forme d'une languette (1) est une paroi de cordon ombilical.

Dans un mode de réalisation, le dispositif selon l'invention est caractérisé en ce que ledit matériau biocompatible sous forme d'une languette (1) est stérile.

Dans un mode de réalisation, le dispositif selon l'invention est caractérisé en ce que ledit matériau biocompatible sous forme d'une languette (1) est viro-inactivé.

On entend par « viro-inactivation », une technique qui permet de réduire fortement ou totalement, et définitivement, la capacité d'action des virus. Ceux-ci, définis comme inactivés, perdent leurs capacités pathogéniques et de réplication par une diminution de leur population de 4 log lors des titrages résiduels qui suivent une ou deux étapes chimiques indépendantes, que ce soit sur des virus enveloppés, ou non enveloppés, ADN ou ARN.

Un tel procédé est par exemple décrit dans le document WO2017140914.

Dans un mode de réalisation, le dispositif selon l'invention est caractérisé en ce que ledit matériau biocompatible sous forme d'une languette (1) est viro-inactivé selon les deux étapes de viro-inactivation chimique du procédé décrit dans WO2017140914.

Dans un mode de réalisation, le dispositif selon l'invention est caractérisé en ce que ledit matériau biocompatible sous forme d'une languette (1) est lyophilisé.

On entend par « lyophilisation », une technique visant à dessécher un produit préalablement surgelé par sublimation. Plus précisément, le liquide à ôter du produit est dans un premier temps transformé en glace par congélation ; puis par une dessiccation primaire, sous vide, la glace est sublimée ; enfin par une dessiccation secondaire, les molécules d 'eau à la surface du produit sont extraites par désorption.

Dans un mode de réalisation, le dispositif selon l'invention est caractérisé en ce que ledit matériau biologique issu de cordon ombilical sous forme d'un broyat moulé et formant un manchon (4) a une section de forme géométrique choisie dans le groupe constitué des formes de carré, de triangle, de cercle, de rectangle, d'octogone, de losange, de trapèze, d'ovale, d'ellipse, de pentagone et d'hexagone.

On entend par « section », une forme géométrique plane formée par un plan orthogonal à l'axe du manchon.

Le manchon (4) aura une section de diamètre équivalent adapté à la largeur de la fistule à combler, ainsi qu'une longueur adaptée à la longueur de la fistule à combler.

Dans un mode de réalisation, le dispositif selon l'invention est caractérisé en ce que ledit matériau biologique issu de cordon ombilical sous forme d'un broyat moulé et formant un manchon (4) a une longueur comprise entre 1 et 20 cm, de préférence entre 1 et 4,5 cm.

Dans un mode de réalisation, le dispositif selon l'invention est caractérisé en ce que ledit matériau biologique issu de cordon ombilical sous forme d'un broyat moulé et formant un manchon (4) a une section de diamètre équivalent compris entre 0,1 et 5 cm, de préférence entre 0,4 et 1 cm.

Dans un mode de réalisation, le dispositif selon l'invention est caractérisé en ce que ledit matériau biologique issu de cordon ombilical sous forme d'un broyat moulé et formant un manchon (4) a une section de surface comprise entre 0,005 et 20 cm², de préférence entre 0,1 et 1 cm².

Le matériau biologique issu de cordon ombilical a pour but de permettre la régénération du tissu de la fistule.

Dans un mode de réalisation, le dispositif selon l'invention est caractérisé en ce que ledit matériau biologique issu de cordon ombilical sous forme d'un broyat moulé et formant un manchon (4) est de la gelée de Wharton.

On entend par « gelée de Wharton », le tissu biologique gélatineux présent dans le cordon ombilical des mammifères dont la veine et les deux artères naturellement incluses au sein dudit tissu biologique gélatineux ont été retirées. Dans la présente invention, le terme « gelée de Wharton » peut être entendu comme comprenant ou non la membrane amniotique entourant la gelée de Wharton du cordon ombilical. Dans la présente invention, le terme « gelée de Wharton » est entendu comme ne comprenant pas de fibres de collagène épaisses et/ou de lacunes et parois vasculaires (villosités et chambres intervilleuses).

Dans un mode de réalisation, le dispositif selon l'invention est caractérisé en ce que ledit matériau biologique issu de cordon ombilical sous forme d'un broyat moulé et formant un manchon (4) comprend au moins un principe actif.

Dans un mode de réalisation, le dispositif selon l'invention est caractérisé en ce que ledit matériau biologique issu de cordon ombilical sous forme d'un broyat moulé et formant un manchon (4) comprend au moins un principe actif ajouté par imprégnation.

Dans un mode de réalisation, le dispositif selon l'invention est caractérisé en ce que ledit matériau biologique issu de cordon ombilical sous forme d'un broyat moulé et formant un manchon (4) comprend au moins un principe actif choisi dans le groupe constitué des antibiotiques, des antiseptiques, des antiviraux, des anticorps monoclonaux, des inhibiteurs semi-synthétiques des métalloprotéases, des agents immunosuppresseurs, des anti-inflammatoires, des antifongiques, des anti-allergiques, des anesthésiques, ou des protéines immunoadhésives, seuls ou en combinaisons.

Dans un mode de réalisation, le dispositif selon l'invention est caractérisé en ce que ledit matériau biologique issu de cordon ombilical sous forme d'un broyat moulé et formant un manchon (4) comprend au moins un principe actif choisi dans le groupe constitué des antibiotiques. Quelques exemples d'antibiotiques utilisables sont donnés ci-après : tétracyclines (daunomycine, tétracycline, chloretetracycline, oxytétracycline, etc.), glycopeptides (vancomycine, etc.), aminoglycosides (gentamycine, etc.), aminosides (tobramycine, néomycine, etc.), fluoroquinolones (ciprofloxacine, moxifloxacin, etc.), quinolones (gatifloxacine, etc.), polypeptides (bacitracine, polymyxine, etc.), phénicolés (chloramphénicol, etc.), macrolides (erythromycine, etc.), sulfonamides (sulfacétamide, sulfaméthoxazole, sulfisoxazole, etc.), céphalosporines (céfradoxil, céfoxitine, etc.), et tout autre antibiotique.

Dans un mode de réalisation, le dispositif selon l'invention est caractérisé en ce que ledit matériau biologique issu de cordon ombilical sous forme d'un broyat moulé et formant un manchon (4) comprend au moins un principe actif choisi dans le groupe constitué des anti-inflammatoires stéroïdiens (AIS) et/ou non-stéroïdiens (AINS). Quelques exemples d'AIS sont donnés ci-après : triamcinolone, dexaméthasone, prednisolone, hydrocortisone, corticostérone, fluocinolone, prednisolone, méthylprednisolone, fluorométholone, betaméthasone, tétrahydrocortisol, riméxolone, etc. Quelques exemples d'AINS sont donnés ci-après : indométacine, népafénac, diclofénac, bromfénac, kétorolac, suprofène, etc.

Dans un mode de réalisation, le dispositif selon l'invention est caractérisé en ce que ledit matériau biologique issu de cordon ombilical sous forme d'un broyat moulé et formant un manchon (4) comprend au moins un principe actif choisi dans le groupe constitué des immunosuppresseurs, une liste non-limitative est donnée ci-après : déxaméthasone, bétaméthasone, etc.

Dans un mode de réalisation, le dispositif selon l'invention est caractérisé en ce que ledit matériau biologique issu de cordon ombilical sous forme d'un broyat moulé et formant un manchon (4) comprend au moins un principe actif choisi dans le groupe constitué des antiviraux, une liste non-limitative est donnée ci-après : ganciclovir, trifluorothymidine, aciclovir, DDI, AZT, foscarnet, vidarabine, trifluorouridine, idoxuridine, ribavirine, inhibiteurs de protéase, agent anti-cytomégalovirus, etc.

Dans un mode de réalisation, le dispositif selon l'invention est caractérisé en ce que ledit matériau biologique issu de cordon ombilical sous forme d'un broyat moulé et formant un manchon (4) comprend au moins un principe actif choisi dans le groupe constitué des antifongiques, une liste non-limitative est donnée ci-après : fluconazole, nitrofurazone, amphotéricine B, kétoconazole, etc.

Dans un mode de réalisation, le dispositif selon l'invention est caractérisé en ce que ledit matériau biologique issu de cordon ombilical sous forme d'un broyat moulé et formant un manchon (4) comprend au moins un principe actif choisi dans le groupe constitué des antiallergiques, une liste non-limitative est donnée ci-après : méthapyriline, chlorpheniramine, pyrilamine, prophenpyridamine, etc.

Dans un mode de réalisation, le dispositif selon l'invention est caractérisé en ce que ledit matériau biologique issu de cordon ombilical sous forme d'un broyat moulé et formant un manchon (4) comprend au moins un principe actif choisi dans le groupe constitué des anesthésiques, une liste non-limitative est donnée ci-après : lidocaïne, mépivacaïne, etc.

Dans un mode de réalisation, le dispositif selon l'invention est caractérisé en ce que ledit matériau biologique issu de cordon ombilical sous forme d'un broyat moulé et formant un manchon (4) est stérile.

Dans un mode de réalisation, le dispositif selon l'invention est caractérisé en ce que ledit matériau biologique issu de cordon ombilical sous forme d'un broyat moulé et formant un manchon (4) est viro-inactivé.

Dans un mode de réalisation, le dispositif selon l'invention est caractérisé en ce que ledit matériau biologique issu de cordon ombilical sous forme d'un broyat moulé et formant un manchon (4) est viro-inactivé selon les deux étapes de viro-inactivation chimique du procédé décrit dans WO2017140914.

Dans un mode de réalisation, le dispositif selon l'invention est caractérisé en ce que ledit matériau biologique issu de cordon ombilical sous forme d'un broyat moulé et formant un manchon (4) est lyophilisé.

Dans un mode de réalisation, le dispositif selon l'invention est caractérisé en ce qu'il est incurvé.

L'invention concerne également un procédé de fabrication d'un dispositif de comblement de fistule comprenant les étapes de :
a) on dipose d'un matériau biocompatible sous forme de languette (1) ayant une extrémité proximale (2) et une extrémité distale (3);
b) on place ledit matériau biocompatible dans un moule de façon à ce que celui-ci traverse le moule ;
c) on rempli le moule d'un matériau biologique issu de cordon ombilical sous forme d'un broyat de façon à ce qu'il entoure ledit matériau biocompatible sous forme de languette formant ainsi un manchon (4);
d) on solidifie ledit matériau biologique issu de cordon ombilical.

Dans un mode de réalisation, le procédé de fabrication d'un dispositif de comblement de fistule selon l'invention est caractérisé en ce que la solidification du matériau biologique issu de cordon ombilical est réalisée par lyophilisation.

Dans un mode de réalisation, le procédé de fabrication d'un dispositif de comblement de fistule selon l'invention est caractérisé en ce que la longueur du moule est inférieure à la longueur dudit matériau biocompatible sous forme de languette (1).

Dans un mode de réalisation, le procédé de fabrication d'un dispositif de comblement de fistule selon l'invention est caractérisé en ce que le moule a une section de forme géométrique choisie dans le groupe constitué des formes de carré, de triangle, de cercle, de rectangle, d'octogone, de losange, de trapèze, d'ovale, d'ellipse, de pentagone et d'hexagone.

Le moule aura une section de diamètre équivalent adapté à la largeur de la fistule à combler, ainsi qu'une longueur adaptée à la longueur de la fistule à combler.

Dans un mode de réalisation, le procédé de fabrication d'un dispositif de comblement de fistule selon l'invention est caractérisé en ce que le moule a une longueur comprise entre 1 et 20 cm, de préférence entre 1 et 4,5 cm.

Dans un mode de réalisation, le procédé de fabrication d'un dispositif de comblement de fistule selon l'invention est caractérisé en ce que le moule a une section de diamètre équivalent compris entre 0,1 et 5 cm, de préférence entre 0,4 et 1 cm.

Dans un mode de réalisation, le procédé de fabrication d'un dispositif de comblement de fistule selon l'invention est caractérisé en ce que le moule a une section de surface comprise entre 0,005 et 20 cm², de préférence entre 0,1 et 1 cm².

Dans un mode de réalisation, le procédé de fabrication d'un dispositif de comblement de fistule selon l'invention est caractérisé en ce que ladite languette (1) possède une extrémité proximale (2) plus large que l'extrémité distale (3).

Dans un mode de réalisation, le procédé de fabrication d'un dispositif de comblement de fistule selon l'invention est caractérisé en ce que l'extrémité proximale (2) dudit matériau biocompatible sous forme de languette (1) est de forme géométrique choisie dans le groupe constitué des formes de carré, de triangle, de cercle, de rectangle, d'octogone, de losange, de trapèze, d'ovale, d'ellipse, de pentagone et d'hexagone.

Dans un mode de réalisation, le procédé de fabrication d'un dispositif de comblement de fistule selon l'invention est caractérisé en ce que ledit matériau biocompatible sous forme d'une languette (1) est un matériau biologique.

Dans un mode de réalisation, le procédé de fabrication d'un dispositif de comblement de fistule selon l'invention est caractérisé en ce que ledit matériau biocompatible sous forme d'une languette (1) est un matériau biologique issu de cordon ombilical.

Dans un mode de réalisation, le procédé de fabrication d'un dispositif de comblement de fistule selon l'invention est caractérisé en ce que ledit matériau biocompatible sous forme d'une languette (1) est une paroi de cordon ombilical.

Dans un mode de réalisation, le procédé de fabrication d'un dispositif de comblement de fistule selon l'invention est caractérisé en ce que ledit matériau biocompatible sous forme d'une languette (1) est stérile.

Dans un mode de réalisation, le procédé de fabrication d'un dispositif de comblement de fistule selon l'invention est caractérisé en ce que ledit matériau biocompatible sous forme d'une languette (1) est viro-inactivé.

Dans un mode de réalisation, le procédé de fabrication d'un dispositif de comblement de fistule selon l'invention est caractérisé en ce que ledit matériau biocompatible sous forme d'une languette (1) est viro-inactivé selon les deux étapes de viro-inactivation chimique du procédé décrit dans WO2017140914.

Dans un mode de réalisation, le procédé de fabrication d'un dispositif de comblement de fistule selon l'invention est caractérisé en ce que ledit matériau biocompatible sous forme d'une languette (1) est lyophilisé.

Dans un mode de réalisation, le procédé de fabrication d'un dispositif de comblement de fistule selon l'invention est caractérisé en ce que ledit matériau biologique issu de cordon ombilical formant un manchon (4) est de la gelée de Wharton.

Dans un mode de réalisation, le procédé de fabrication d'un dispositif de comblement de fistule selon l'invention est caractérisé en ce que ledit matériau biologique issu de cordon ombilical formant un manchon (4) comprend au moins un principe actif.

Dans un mode de réalisation, le procédé de fabrication d'un dispositif de comblement de fistule selon l'invention est caractérisé en ce que ledit matériau biologique issu de cordon ombilical formant un manchon (4) comprend au moins un principe actif ajouté par imprégnation.

Dans un mode de réalisation, le procédé de fabrication d'un dispositif de comblement de fistule selon l'invention est caractérisé en ce que ledit matériau biologique issu de cordon ombilical sous forme d'un broyat moulé et formant un manchon (4) comprend au moins un principe actif choisi dans le groupe constitué des antibiotiques, des antiseptiques, des antiviraux, des anticorps monoclonaux, des inhibiteurs semi-synthétiques des métalloprotéases, des agents immunosuppresseurs, des anti-inflammatoires, des antifongiques, des anti-allergiques, des anesthésiques, ou des protéines immunoadhésives, seuls ou en combinaisons.

Dans un mode de réalisation, le procédé de fabrication d'un dispositif de comblement de fistule selon l'invention est caractérisé en ce que ledit matériau biologique issu de cordon ombilical sous forme d'un broyat moulé et formant un manchon (4) comprend au moins un principe actif choisi dans le groupe constitué des antibiotiques. Quelques exemples d'antibiotiques utilisables sont donnés ci-après : tétracyclines (daunomycine, tétracycline, chloretetracycline, oxytétracycline, etc.), glycopeptides (vancomycine, etc.), aminoglycosides (gentamycine, etc.), aminosides (tobramycine, néomycine, etc.), fluoroquinolones (ciprofloxacine, moxifloxacin, etc.), quinolones (gatifloxacine, etc.), polypeptides (bacitracine, polymyxine, etc.), phénicolés (chloramphénicol, etc.), macrolides (erythromycine, etc.), sulfonamides (sulfacétamide, sulfaméthoxazole, sulfisoxazole, etc.), céphalosporines (céfradoxil, céfoxitine, etc.), et tout autre antibiotique.

Dans un mode de réalisation, le procédé de fabrication d'un dispositif de comblement de fistule selon l'invention est caractérisé en ce que ledit matériau biologique issu de cordon ombilical sous forme d'un broyat moulé et formant un manchon (4) comprend au moins un principe actif choisi dans le groupe constitué des anti-inflammatoires stéroïdiens (AIS) et/ou non-stéroïdiens (AINS). Quelques exemples d'AIS sont donnés ci-après : triamcinolone, dexaméthasone, prednisolone, hydrocortisone, corticostérone, fluocinolone, prednisolone, méthylprednisolone, fluorométholone, betaméthasone, tétrahydrocortisol, riméxolone, etc. Quelques exemples d'AINS sont donnés ci-après : indométacine, népafénac, diclofénac, bromfénac, kétorolac, suprofène, etc.

Dans un mode de réalisation, le procédé de fabrication d'un dispositif de comblement de fistule selon l'invention est caractérisé en ce que ledit matériau biologique issu de cordon ombilical sous forme d'un broyat moulé et formant un manchon (4) comprend au moins un principe actif choisi dans le groupe constitué des immunosuppresseurs, une liste non-limitative est donnée ci-après : déxaméthasone, bétaméthasone, etc.

Dans un mode de réalisation, le procédé de fabrication d'un dispositif de comblement de fistule selon l'invention est caractérisé en ce que ledit matériau biologique issu de cordon ombilical sous forme d'un broyat moulé et formant un manchon (4) comprend au moins un principe actif choisi dans le groupe constitué des antiviraux, une liste non-limitative est donnée ci-après : ganciclovir, trifluorothymidine, aciclovir, DDI, AZT, foscarnet, vidarabine, trifluorouridine, idoxuridine, ribavirine, inhibiteurs de protéase, agent anti-cytomégalovirus, etc.

Dans un mode de réalisation, le procédé de fabrication d'un dispositif de comblement de fistule selon l'invention est caractérisé en ce que ledit matériau biologique issu de cordon ombilical sous forme d'un broyat moulé et formant un manchon (4) comprend au moins un principe actif choisi dans le groupe constitué des antifongiques, une liste non-limitative est donnée ci-après : fluconazole, nitrofurazone, amphotéricine B, kétoconazole, etc.

Dans un mode de réalisation, le procédé de fabrication d'un dispositif de comblement de fistule selon l'invention est caractérisé en ce que ledit matériau biologique issu de cordon ombilical sous forme d'un broyat moulé et formant un manchon (4) comprend au moins un principe actif choisi dans le groupe constitué des antiallergiques, une liste non-limitative est donnée ci-après : méthapyriline, chlorpheniramine, pyrilamine, prophenpyridamine, etc.

Dans un mode de réalisation, le procédé de fabrication d'un dispositif de comblement de fistule selon l'invention est caractérisé en ce que ledit matériau biologique issu de cordon ombilical sous forme d'un broyat moulé et formant un manchon (4) comprend au moins un principe actif choisi dans le groupe constitué des anesthésiques, une liste non-limitative est donnée ci-après : lidocaïne, mépivacaïne, etc.

Dans un mode de réalisation, le procédé de fabrication d'un dispositif de comblement de fistule selon l'invention est caractérisé en ce que ledit matériau biologique issu de cordon ombilical formant un manchon (4) est stérile.

Dans un mode de réalisation, le procédé de fabrication d'un dispositif de comblement de fistule selon l'invention est caractérisé en ce que ledit matériau biologique issu de cordon ombilical formant un manchon (4) est viro-inactivé.

Dans un mode de réalisation, le procédé de fabrication d'un dispositif de comblement de fistule selon l'invention est caractérisé en ce que ledit matériau biocompatible sous forme d'une languette (1) est viro-inactivé selon les deux étapes de viro-inactivation chimique du procédé décrit dans WO2017140914.

Il est également divulgué un procédé de comblement de fistule, ce procédé ne fait pas partie de l'invention. Le procédé comprenant les étapes de :
e) Insertion dans la fistule d'un dispositif selon l'invention.

Dans un mode de réalisation, le procédé de comblement de fistule selon l'invention est caractérisé en ce qu'il comprend une étape ultérieure f) de fixation de l'extrémité proximale (2) du matériau biocompatible sous forme de languette (1) à la paroi du tissu dans lequel est située l'extrémité de la fistule.

Dans un mode de réalisation, le procédé de comblement de fistule selon l'invention est caractérisé en ce que l'étape de fixation de ladite extrémité proximale (2) est réalisée par suture.

Dans un mode de réalisation, le procédé de comblement de fistule selon l'invention est caractérisé en ce que l'étape de fixation de ladite extrémité proximale (2) est réalisée par collage.

Dans un mode de réalisation, le procédé de comblement de fistule selon l'invention est caractérisé en ce que ladite fistule est une fistule choisie dans le groupe constitué des fistules ano-rectales, urètro-vaginales, vesico-vaginales, recto-vaginales, trachéo-esophagiques, gastro-cutanées, recto-vésicales, recto-urètrales, recto-prostatiques.

Dans un mode de réalisation, le procédé de comblement de fistule selon l'invention est caractérisé en ce que ladite fistule a une longueur comprise entre 1 et 20 cm, de préférence entre 1 et 4,5 cm.

Dans un mode de réalisation, le procédé de comblement de fistule selon l'invention est caractérisé en ce que ladite fistule a une largeur comprise entre 0,1 et 5 cm, de préférence entre 0,4 et 1 cm.

### Exemples

### Exemple 1 : Fabrication d'un dispositif de comblement de fistule selon l'invention.

[000139] Un donneur proprement informé et consentant en accord avec les exigences de la Déclaration d'Helsinki offre en don le cordon ombilical issu d'un accouchement. Par exigence sanitaire relative aux dons de tissus et de cellules d'origine humaine, une qualification en amont du donneur est systématique. Cette qualification implique une recherche de virus HIV, hépatites B, C, HTLV et de la bactérie tréponème pâle responsable de la syphilis.

Le cordon ombilical est récupéré le plus tôt possible en salle d'accouchement. Il est avantageusement placé dans une boite stérile, comprenant une solution de NaCl à +4°C.

Au laboratoire, en salle stérile, la procédure suivante est appliquée :
- Un segment de 20 cm à 50 cm de longueur est isolé par section du cordon ombilical.
- Le cordon ombilical est rincé et hydraté dans des bains d'eau purifiée successifs, sous agitation douce, pendant 4 heures.
- Les vaisseaux sanguins du segment de cordon ombilical sont repérés et séparés du reste du segment afin de conserver uniquement la gelée de Wharton et la paroi qui I 'entoure.
- Les parois de cordon sont conservées à sec au congélateur.
- Un morceau de paroi de cordon ombilical traité chimiquement puis lyophilisé est découpé puis mis sous forme d'une languette de dimensions (longueur et largeur) appropriées pour la fistule à combler.
- La gelée de Wharton après avoir subi un traitement chimique de viro-inactivation est insérée dans un broyeur vibrant à billes Retsch MM400, équipé avec un bol en oxyde de zirconium de 35 mL. La gelée de Wharton occupe un espace d'environ 1/3 du bol. Une bille en oxyde de zirconium d'un diamètre de 20 mm est ajoutée dans le bol avec la gelée de Wharton. Un broyage de 1 minute est effectué à une fréquence de 3 Hz. La bille d'un diamètre de 20 mm est récupérée, et 9 billes en oxyde de zirconium d'un diamètre de 10 mm sont ajoutées dans le bol. Un deuxième broyage de 3 minutes est réalisé à une fréquence de 30 Hz. Un troisième broyage est effectué avec 60 billes de 5mm pendant 3 minutes à une fréquence de 30 Hz.
- La languette lyophilisée est insérée dans un moule puis la gelée de Wharton broyée est introduite dans le moule de façon à ce que la gelée entoure la languette formant ainsi un manchon.
- L'ensemble est ensuite lyophilisé afin que la gelée de Wharton se solidifie.

Est ainsi obtenu un dispositif de comblement de fistule constituée d'une languette de paroi de cordon ombilical entourée de gelée de Wharton.

Le dispositif est enfin stérilisé.

## Revendications

1. Dispositif de comblement de fistule comprenant :
- un matériau biocompatible sous forme d'une languette (1) ayant une extrémité proximale (2) et une extrémité distale (3);
- un matériau biologique sous forme d'un broyat moulé entourant le matériau biocompatible et formant un manchon (4) ;
**caractérisé en ce que** ledit matériau biologique est issu de cordon ombilical.

2. Dispositif de comblement de fistule selon la revendication 1, **caractérisé en ce que** le matériau biocompatible sous forme d'une languette (1) possède une longueur supérieure à celle du manchon (4).

3. Dispositif de comblement de fistule selon la revendication 2, **caractérisé en ce que** l'extrémité proximale (2) de ladite languette (1) n'est pas entourée par le manchon (4).

4. Dispositif de comblement de fistule selon la revendication 3, **caractérisé en ce que** l'extrémité proximale (2) de ladite languette (1) est plus large que l'extrémité distale (3).

5. Dispositif de comblement de fistule selon l'une quelconque des revendications 2 à 4, **caractérisé en ce que** l'extrémité distale (3) de ladite languette (1) n'est pas entourée du manchon (4).

6. Dispositif de comblement de fistule selon l'une quelconque des revendications précédentes, **caractérisé en ce que** le matériau biocompatible sous forme d'une languette (1) a une longueur comprise entre 1 et 20 cm, de préférence entre 2 et 10 cm.

7. Dispositif de comblement de fistule selon l'une quelconque des revendications 1 à 5 **caractérisé en ce que** ledit matériau biologique issu de cordon ombilical sous forme d'un broyat moulé et formant un manchon (4) a une longueur comprise entre 1 et 20 cm, de préférence entre 1 et 4,5 cm.

8. Dispositif de comblement de fistule selon la revendication 1, **caractérisé en ce que** le matériau biocompatible sous forme d'une languette (1) a une longueur comprise entre 1 et 20 cm, de préférence entre 2 et 10 cm et que ledit matériau biologique issu de cordon ombilical sous forme d'un broyat moulé et formant un manchon (4) a une longueur comprise entre 1 et 20 cm, de préférence entre 1 et 4,5 cm.

9. Dispositif de comblement de fistule selon l'une quelconque des revendications précédentes, **caractérisé en ce que** ledit matériau biocompatible sous forme d'une languette (1) est une paroi de cordon ombilical.

10. Dispositif de comblement de fistule selon l'une quelconque des revendications précédentes, **caractérisé en ce que** ledit matériau biologique issu de cordon ombilical sous forme d'un broyat moulé et formant un manchon (4) est de la gelée de Wharton.

11. Procédé de fabrication d'un dispositif de comblement de fistule comprenant les étapes de :
a) on dipose d'un matériau biocompatible sous forme de languette (1) ayant une extrémité proximale (2) et une extrémité distale (3);
b) on place ledit matériau biocompatible dans un moule de façon à ce que celui-ci traverse le moule ;
c) on rempli le moule d'un matériau biologique issu de cordon ombilical sous forme d'un broyat de façon à ce qu'il entoure ledit matériau biocompatible sous forme de languette formant ainsi un manchon (4);
d) on solidifie ledit matériau biologique.

12. Procédé de fabrication d'un dispositif de comblement de fistule selon la revendication 11, **caractérisé en ce que** la solidification du matériau biologique issu de cordon ombilical est réalisée par lyophilisation.

13. Procédé de fabrication d'un dispositif de comblement de fistule selon l'une quelconque des revendications 11 et 12, **caractérisé en ce que** ledit matériau biocompatible sous forme d'une languette (1) est une paroi de cordon ombilical.

14. Procédé de fabrication d'un dispositif de comblement de fistule selon l'une quelconque des revendications 11 à 13, **caractérisé en ce que** ledit matériau biologique issu de cordon ombilical formant un manchon (4) est de la gelée de Wharton

## Patentansprüche

1. Vorrichtung zum Auffüllen von Fisteln, umfassend:
- ein biokompatibles Material in Form einer Lasche (1) mit einem proximalen Ende (2) und einem distalen Ende (3);
- ein biologisches Material in Form eines geformten Mahlguts, das das biokompatible Material umgibt und eine Hülse (4) bildet;
**dadurch gekennzeichnet, dass** das biologische Material aus Nabelschnur stammt.

2. Vorrichtung zum Auffüllen von Fisteln nach Anspruch 1, **dadurch gekennzeichnet, dass** das biokompatible Material in Form einer Lasche (1) eine größere Länge als die Hülse (4) besitzt.

3. Vorrichtung zum Auffüllen von Fisteln nach Anspruch 2, **dadurch gekennzeichnet, dass** das proximale Ende (2) der Lasche (1) nicht von der Hülse (4) umgeben ist.

4. Vorrichtung zum Auffüllen von Fisteln nach Anspruch 3, **dadurch gekennzeichnet, dass** das proximale Ende (2) der Lasche (1) breiter ist als das distale Ende (3).

5. Vorrichtung zum Auffüllen von Fisteln nach einem der Ansprüche 2 bis 4, **dadurch gekennzeichnet, dass** das distale Ende (3) der Lasche (1) nicht von der Hülse (4) umgeben ist.

6. Vorrichtung zum Auffüllen von Fisteln nach einem der vorhergehenden Ansprüche, **dadurch gekennzeichnet, dass** das biokompatible Material in Form einer Lasche (1) eine Länge zwischen 1 und 20 cm, bevorzugterweise zwischen 2 und 10 cm, aufweist.

7. Vorrichtung zum Auffüllen von Fisteln nach einem der Ansprüche 1 bis 5, **dadurch gekennzeichnet, dass** das biologische Material aus Nabelschnur in Form eines geformten Mahlguts, das eine Hülse (4) bildet, eine Länge zwischen 1 und 20 cm, bevorzugterweise zwischen 1 und 4,5 cm, aufweist.

8. Vorrichtung zum Auffüllen von Fisteln nach Anspruch 1, **dadurch gekennzeichnet, dass** das biokompatible Material in Form einer Lasche (1) eine Länge zwischen 1 und 20 cm, bevorzugterweise zwischen 2 und 10 cm, aufweist, und dass das biologische Material aus Nabelschnur in Form eines geformten Mahlgutes, das eine Hülse (4) ausbildet, eine Länge zwischen 1 und 20 cm, bevorzugterweise zwischen 1 und 4,5 cm, aufweist.

9. Vorrichtung zum Füllen einer Fistel nach einem der vorhergehenden Ansprüche, **dadurch gekennzeichnet, dass** das biokompatible Material in Form einer Lasche (1) eine Nabelschnurwand ist.

10. Vorrichtung zum Füllen einer Fistel nach einem der vorhergehenden Ansprüche, **dadurch gekennzeichnet, dass** das aus Nabelschnur stammende biologische Material in Form eines eine Hülse (4) ausbildenden geformten Mahlguts Whartons Gelee ist.

11. Verfahren zum Herstellen einer Vorrichtung zum Auffüllen einer Fistel, das die folgenden Schritte umfasst:
a) Bereitstellen eines biokompatiblen Materials in Form einer Lasche (1) mit einem proximalen Ende (2) und einem distalen Ende (3);
b) Anordnen des biokompatiblen Materials in einer Form, so dass es die Form durchdringt;
c) Befüllen der Form mit einem aus Nabelschnur stammenden biologischen Material in Form eines Mahlguts, so dass es das biokompatible Material in Form einer Lasche umgibt und so eine Hülse (4) bildet;
d) Verfestigen des biologischen Materials.

12. Verfahren zum Herstellen einer Vorrichtunug zum Füllen einer Fistel nach Anspruch 11, **dadurch gekennzeichnet, dass** das Verfestigen des aus Nabelschnur stammenden biologischen Materials durch Lyophilisieren erfolgt.

13. Verfahren zum Herstellen einer Vorrichtunug zum Füllen einer Fistel nach einem der Ansprüche 11 oder 12, **dadurch gekennzeichnet, dass** das biokompatible Material in Form einer Lasche (1) eine Nabelschnurwand ist.

14. Verfahren zum Herstellen einer Vorrichtunug zum Füllen einer Fistel nach einem der Ansprüche 11 bis 13, **dadurch gekennzeichnet, dass** das aus Nabelschnur stammende biologische Material, das eine Hülse (4) bildet, Whartons Gelee ist.

## Claims

1. Fistula filling device comprising:
- a biocompatible material in the form of a tab (1) having a proximal end (2) and a distal end (3);
- a biological material in the form of a molded ground material surrounding the biocompatible material and forming a sleeve (4);
**characterized in that** said biological material is derived from the umbilical cord.

2. Fistula filling device according to claim 1, **characterized in that** the biocompatible material in the form of a tab (1) has a length greater than that of the sleeve (4).

3. Fistula filling device according to claim 2, **characterized in that** the proximal end (2) of said tab (1) is not surrounded by the sleeve (4).

4. Fistula filling device according to claim 3, **characterized in that** the proximal end (2) of said tab (1) is wider than the distal end (3).

5. Fistula filling device according to any one of claims 2 to 4, **characterized in that** the distal end (3) of said tab (1) is not surrounded by the sleeve (4).

6. Fistula filling device according to any one of the preceding claims, **characterized in that** the biocompatible material in the form of a tab (1) has a length of between 1 and 20 cm, preferably between 2 and 10 cm.

7. Fistula filling device according to any one of claims 1 to 5, **characterized in that** said biological material from the umbilical cord in the form of a molded ground material and forming a sleeve (4) has a length of between 1 and 20 cm, preferably between 1 and 4.5 cm.

8. Fistula filling device according to claim 1, **characterized in that** the biocompatible material in the form of a tab (1) has a length of between 1 and 20 cm, preferably between 2 and 10 cm and that said biological material from the umbilical cord in the form of a molded ground material and forming a sleeve (4) has a length of between 1 and 20 cm, preferably between 1 and 4.5 cm.

9. Fistula filling device according to any one of the preceding claims, **characterized in that** said biocompatible material in the form of a tab (1) is an umbilical cord wall.

10. Fistula filling device according to any one of the preceding claims, **characterized in that** said biological material from the umbilical cord in the form of a molded ground material and forming a sleeve (4) is Wharton's jelly.

11. Method of manufacturing a fistula filling device comprising the following steps:
a) a biocompatible material in the form of a tab (1) is provided having a proximal end (2) and a distal end (3);
b) said biocompatible material is placed in a mold so that it passes through the mold;
c) the mold is filled with a biological material originating from the umbilical cord in the form of ground material so as to surround said biocompatible material in the form of a tab, thus forming a sleeve (4);
(d) said biological material is solidified.

12. Method of manufacturing a fistula filling device according to claim 11, **characterized in that** the solidification of the biological material from the umbilical cord is carried out by freeze-drying.

13. Method of manufacturing a fistula filling device according to any one of claims 11 and 12, **characterized in that** said biocompatible material in the form of a tab (1) is an umbilical cord wall.

14. Method of manufacturing a fistula filling device according to any one of claims 11 to 13, **characterized in that** said biological material from the umbilical cord forming a sleeve (4) is Wharton's jelly.
